# EUROPEAN PATENT APPLICATION

(11) **EP 2 438 959 A1**
(43) Date of publication of application: **11.04.2012**
(21) Application number: 11184052.6
(22) Date of filing: 06.10.2011
(51) Int. Cl.: A61N 1/372

(54) **Implantable medical device**

(30) Priority: 07.10.2010 KR 20100097955; 14.01.2011 KR 20110004263
(71) Applicant: Samsung Electronics Co., Ltd., Gyeonggi-do 443-742 (KR)
(72) Inventor: Jung, Myung-June, Suwon-si Gyeonggi-do (KR); Suh, Sang-Bum, Seoul (KR)
(74) Representative: Grünecker, Kinkeldey, Stockmair & Schwanhäusser

(57) **Abstract**

An implantable medical device (IMD) is provided. The IMD includes a medical procedure performing unit configured to perform a medical procedure inside a body of a user, a code generating unit configured to detect a motion state of the user, and to generate a motion code that represents a user's intention based on the detected motion state, and a control unit configured to control the medical procedure of the medical procedure performing unit based on the generated motion code.

## Description

### CROSS-REFERENCE TO RELATED APPLICATION(S)

This application claims the benefit under 35 U.S.C. §119(a) of Korean Patent Application Nos. 10-2010-0097955, filed on October 7, 2010, and 10-2011-0004263, filed on January 14, 2011, in the Korean Intellectual Property Office.

### BACKGROUND

### 1. Field

The following description relates to an implantable medical device (IMD) and method of controlling the same.

### 2. Description of the Related Art

An implantable medical device (IMD) may be implanted into a human body and may operate for a medical purpose.

IMD may monitor a state of primary organs such as the heart and may control the organs to function normally.

In addition, the IMD may provide physiological/pathological state information which may be required for medical practice in, for example, a consulting room, an emergency room, and heath care center.

However, it may be difficult to control or change operation of the IMD because the IMD is implanted in a human body.

Moreover, since the physiological/pathological state information of a patient, which may be provided by the IMD, is substantially private, the provision of such state information to an improper device may result in violating the privacy of the patient.

### SUMMARY

The following description relates to an implantable medical device (IMD) which may be controlled based on a user's intention, and a method of controlling the IMD.

In addition, an IMD is provided that controls access by an external device based on a user's intention and a method of controlling the access.

In one general aspect, an implantable medical device (IMD) is provided. The IMD includes a medical procedure performing unit configured to perform a medical procedure inside a body of a user, a code generating unit configured to detect a motion state of the user, and to generate a motion code that represents a user's intention based on the detected motion state, and a control unit configured to control the medical procedure of the medical procedure performing unit based on the generated motion code.

The medical procedure performing unit may collect physiological/pathological state information of the user.

The control unit may determine whether to transmit the physiological/pathological state information to an external device based on the motion code.

The code generating unit may detect the motion state that includes a standstill state of the user, a moving state of the user, a moving state of user's pupils, a brainwave state of the user, or a combination thereof.

The code generating unit may include a first sensor configured to set a reference for the motion state of the user, and a second sensor configured to detect the motion state which includes the standstill state of the user, a moving state of the user, a moving state of the user's pupils, or a combination thereof.

The first sensor or the second sensor may be placed outside of the user's body to detect a motion state of the user.

The code generating unit may comprise a sensor configured to be worn on or implanted in a user's finger to detect the motion state of the user.

The IMD may further include an authenticating unit configured to authenticate whether the external device which communicates with the IMD is authorized to acquire the physiological/pathological state information of the user.

The code generating unit generates the motion code in response to the authenticating unit authenticating the external device to be authorized to acquire the physiological/pathological state information.

The IMD may further include a notice and alarm generator configured to generate a signal to notify of an access of the external device in response to the external device attempting to access the IMD to communicate therewith.

The notice and alarm generator may generate a recognizable signal in the form of light, sound, vibration, stimulus, or a combination thereof.

The user's intention may include allowing the performance of the medical procedure or restricting the performance of the medical procedure.

In another general aspect, an implantable medical device (IMD) is provided. The IMD includes an information collecting unit configured to collect physiological/pathological state information of a user, and a provision determining unit configured to detect a motion state of the user, and to determine whether to provide the physiological/pathological state information to an external device according to a user's intention identified based on the detected motion state.

The provision determining unit may include a communication module configured to transmit the physiological/pathological state information to the external device, a detecting unit configured to detect a motion state which includes a standstill state of the user, a moving state of the user, a moving state of user's pupils, a brainwave state of the user or a combination thereof, a code generating unit configured to generate a motion code that represents the user's intention for the providing of the physiological/pathological state information based on the detected motion state, and a control unit configured to control the communication module based on the generated motion code.

The IMD may further include a notice and alarm generator configured to generate a signal to notify of an access of the external device in response to the external device attempting to access the IMD to acquire the physiological/pathological state information.

The IMD may further include an authenticating unit configured to authenticate whether the external device that communicates with the IMD is authorized to acquire the physiological/pathological state information.

The authenticating unit may authenticate the external device by determining whether the external device has previously accessed or whether physiological/pathological state information to be transmitted is the same as the physiological/pathological state information requested by the external device.

The provision determining unit may detect the motion state of the user in response to the external device being authorized to acquire the physiological/pathological state information.

The code generating unit may compare the motion state of the user with a previously stored motion state of the user and may generate a motion code corresponding to the detected motion state.

In another general aspect, a method of controlling an implantable medical device (IMD) with respect to an access of an external device is provided. The method includes receiving a request for state information of the IMD or physiological/pathological state information of a user from the external device, identifying a user's intention by detecting a motion state of the user, and determining whether to transmit the requested state information to the external device according to the identified user's intention.

The identifying of the user's intention may include detecting the motion state of the user which includes a standstill state of the user, a moving state of the user, a moving state of user's pupils, a brainwave state of the user, or a combination thereof.

The identifying of the user's intention may include generating a motion code that represents a user's intention based on the detected motion state of the user, and identifying the user's intention that corresponds to the generated motion code by comparing the generated motion code with a previously stored motion code.

The determining whether to transmit the requested state information comprises providing the state information to the external device or transmitting a refusal response to the external device according to the identified user's intention.

In yet another general aspect, an external device for communicating with an implantable medical device (IMD) is provided. The external device includes a communications unit configured to access state information stored in the IMD, and a reception unit configured to receive the state information determined by a motion state of the user corresponding to a user's s intention detected by the IMD.

The user's intention may include allowing the transmission of the state information or restricting the transmission of the state information.

Other features and aspects may be apparent from the following detailed description, the drawings, and the claims.

### BRIEF DESCRIPTION OF THE DRAWINGS

FIG. 1 is a diagram illustrating an example embodiment showing a concept of controlling an implantable medical device based on user's intention.
FIG. 2 is a diagram illustrating an example embodiment of an implantable medical device (IMD).
FIG. 3 is a diagram illustrating another example embodiment of the IMD.
FIG. 4 is a flowchart illustrating an example embodiment of a method of controlling an implantable medical device (IMD) when an external device accesses to the IMD.
FIG. 5 is a diagram illustrating another example embodiment of the IMD.
FIG. 6 is a flowchart illustrating another example embodiment of a method of controlling the IMD when an external device accesses the IMD.
FIG. 7 is a diagram illustrating an example embodiment of an IMD implanted in a human body.
FIGS. 8A and 8B are diagrams illustrating example embodiments of a motion sensor of an IMD.
FIGS. 9A and 9B are diagrams illustrating example embodiments for explaining motion codes generated by a plurality of motion sensors of an IMD.
FIG. 10 is a flowchart illustrating an example embodiment of a method of controlling an IMD having a motion sensor.
FIG. 11 is a flowchart illustrating an example embodiment of a method of controlling the IMD having a motion sensor.
FIG. 12 is a diagram illustrating an example embodiment showing body parts of a user that allows the recognition of a user's intention through a motion state of the user.
FIG. 13 is a diagram illustrating an example embodiment of a structure for controlling an IMD by recognizing a user's intention through the direct detection of the brain activity.
FIG. 14 is a flowchart illustrating an example embodiment of a method of controlling an implantable medical device (IMD) by recognizing a user's intention based on direct detection of brain activity.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and a computer-readable medium having instructions that, when performed by a processor, cause the processor to perform the steps of the methods, apparatuses, and/or systems described herein will be suggested to those of ordinary skill in the art. Also, descriptions of well-known functions and constructions may be omitted for increased clarity and conciseness.

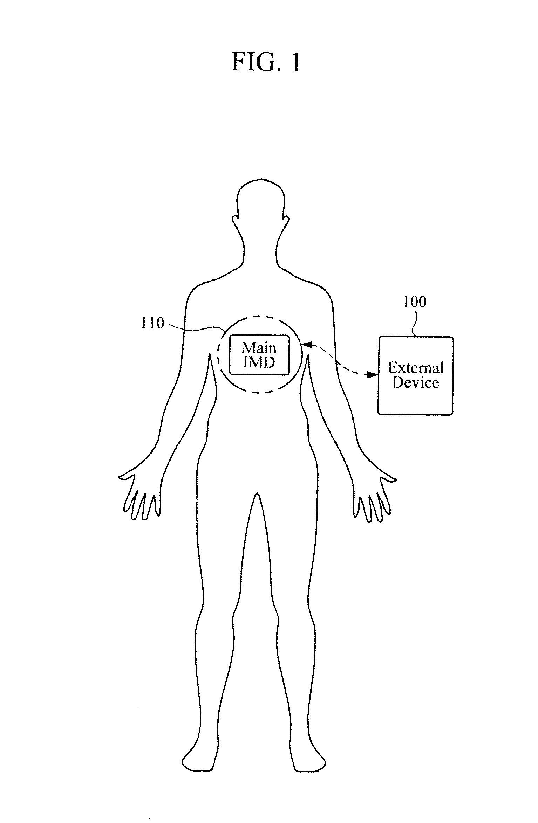

FIG. 1 illustrates a concept of controlling an implantable medical device based on a user's intention.

Referring to FIG. 1, a user may directly control a main implantable medical device (IMD) 110 to perform a variety of operations. For example, the user may directly control the main IMD 110 when an external device 100 makes an access to the main IMD 110 to access state information of the IMD 110, physiological/pathological state information of the user, or other information collected by the IMD 110.

The main IMD 110, which is to be implanted in a body of the user, may collect physiological/pathological information of the user, and may perform a medical procedure to control the operation of an organ while maintaining a close connection with the organ. These are illustrative functions to be provided by an IMD.

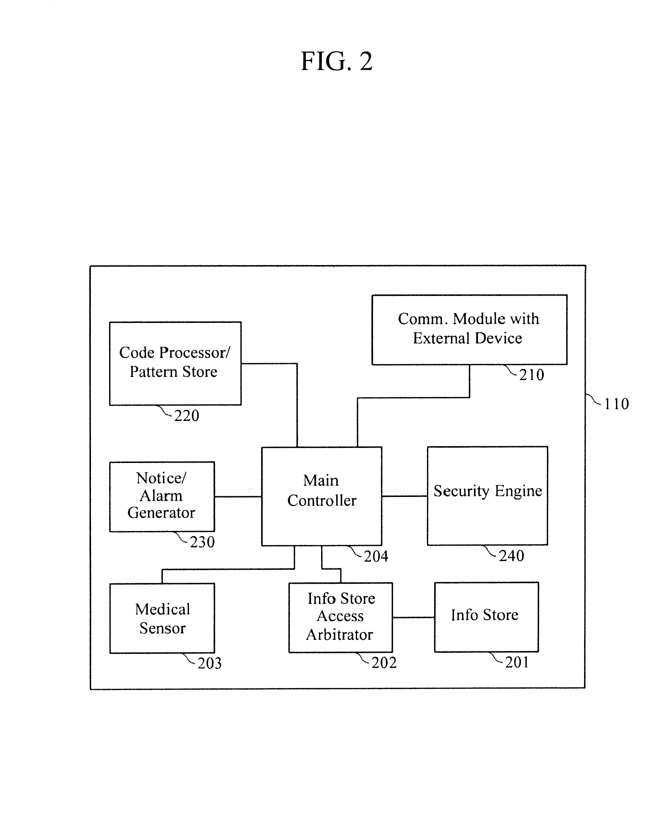

The main IMD 110 may be configured as shown in an example embodiment illustrated in FIG. 2.

FIG. 2 illustrates an example embodiment of an implantable medical device (IMD).

An information store (represented as "Info Store" in FIG. 2) 201 may store state information of the IMD 110, physiological/pathological state information of a user, and other information collected by the IMD 110.

An information store access arbitrator (represented as "Info Store Access Arbitrator" in FIG. 2) 202 may arbitrate attempts to access the information store 201, and log details of all notable accesses of the information store 201.

A medical sensor 203 may collect the physiological/pathological state information of the user, and may, if necessary, perform a medical procedure to control the organ.

A main controller 204 may arbitrate and control functional modules included in the IMD 110.

The above described configuration 201 to 204 may perform basic functions as the IMD may have.

The above configuration, as elements of the IMD, may be represented as below.

A medical procedure performing unit (not shown) may perform a medical procedure inside the body of the user, and may include the medical sensor 203.

A control unit may control the medical procedure of the medical procedure performing unit, and may perform some functions of the main controller 204.

An information collecting unit may collect the physiological/pathological state information of the user, and may perform some functions of the medical sensor 204.

A provision determining unit (not shown) may detect a motion state of the user, and may determine whether to transmit the physiological/pathological state information of the user to an external device based on user's desire based on the detected motion state. The provision determining unit may perform some functions of the main controller 204.

In addition, the IMD 110 may further include a communication module (represented as "Comm Module" in FIG. 2) 210, a code generating unit (hereinafter, it will be referred to as a "code processor/pattern store") 220, a notice and alarm generator 230, and an authenticating unit (hereinafter, it will be referred to as a "security engine") 240.

Each element will be described.

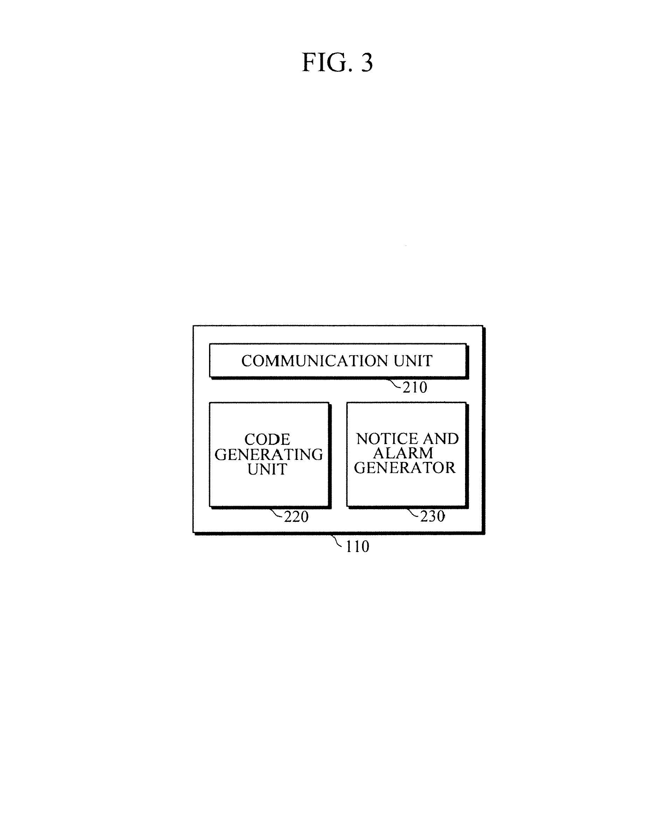

FIG. 3 illustrates another example embodiment of the IMD. Referring to the example embodiment illustrated in FIG. 3, the IMD 110 may include a communication module 210, a code processor/pattern store 220, and notice and alarm generator 230.

The IMD 110, which may be implanted in a human body to collect physiological/pathological state information of a user, to perform a medical procedure to normalize biological activities in the user's body and to communicate with the external device 100 (see FIG. 1), may include the communication module 210 for the communication with the external device 100.

Referring to the example embodiment illustrated in FIG. 1, the external device 100 1) may be connected with the communication module 210 to acquire state information of the IMD 110, 2) may access the IMD 110 to acquire the stored physiological/pathological state information of the user or 3) other information collected by the IMD 110.

The code processor/pattern store 220 may generate a motion code to control the operations including collection of the physiological/pathological state information of the user or the medical procedure to normalize the biological activities in the body. The motion code may be generated based on a detected motion state of the user who is implanted with the IMD 110.

Here, the detected motion state of the user may include almost all movements (such as gestures and motions) which involve changes in user's body. The movements include, for example, standstill or moving of the body, and moving of the pupils. The detected motion state may include any data, for example, brainwaves, which are useful to infer an intention or thinking of the user.

The notice and alarm generator 230 may generate a signal to be transmitted to the user to notify that the IMD 110 is in a state capable of detecting the user's motion state. In other words, the notice and alarm generator 230 may generate a signal to notify the user of an event that the external device 100 attempts access to the IMD 110. In this case, the signal may be in any form including light, sound, vibration, and any kind of stimulus, that the user may recognize. In addition, the signal may be implemented as, for example, a clock, a terminal, or an element of an external device, so the user can recognize the signal.

Thus, the communication module 210 may communicate with the external device 100 to be provided with the state information and the user's physiological/pathological state information from the IMD 110. The notice and alarm generator 230 may generate a signal to notify the user of an access of the external device 100 in response to the IMD 110 receiving a request for the state information from the external device 100.

The user may recognize the access of the external device 100 based on the signal generated by the notice and alarm generator 230, and the code processor/pattern store 220 may detect the motion state of the user, may generate the motion code for identifying a user's intention, and may, if necessary, store the generated motion code. The IMD 110 may perform a specific operation including the providing of state information to the external device 100 and the conducting of a medical procedure inside the user's body based on the motion code.

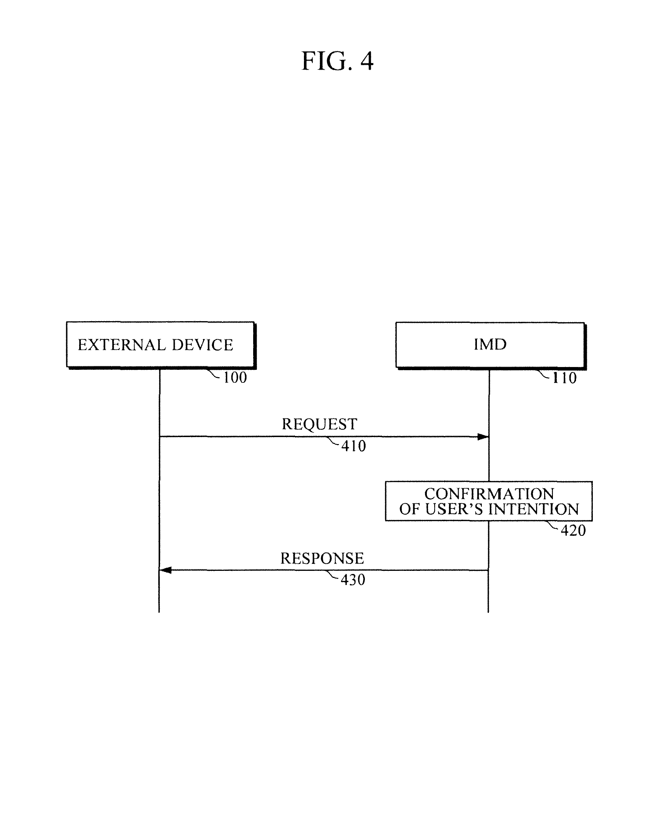

FIG. 4 illustrates a flowchart of an example embodiment of a method of controlling an implantable medical device (IMD) when an external device accesses to the IMD.

The method shown in the example embodiment illustrated in FIG. 4 may be implemented between the external device 100 and the IMD 110 based on their basic configurations.

First, the external device 100 may request the IMD 110, which may retain state information of itself or physiological/pathological state information of a user, to provide such information (410) to the external device.

In response to the request, the IMD 110 may confirm with the user whether to provide the requested information to the external device 100 (420). In response to the user making a movement to allow the IMD 110 to recognize user's intention, the IMD 110 may identify the user's intention.

The code processor/pattern store 220 may detect the motion state of the user which includes a standstill state and a moving state of the user and a moving state of user's pupils, and may generate the motion code based on the motion state. The user's intention may be inferred by comparing the generated motion code and a motion code previously stored in the IMD 110.

The user's intention may involve approving or rejecting the providing of information, and executing operations of the IMD 110, for example, controlling the medical procedure performed by the IMD 110. In addition, the user's intention may be stored or updated as a motion code in the IMD 110.

Then, the IMD 110 may provide state information requested by the external device 100 or transmit a rejection message to the external device 100 based on the user's intention which has been confirmed at 420 (430).

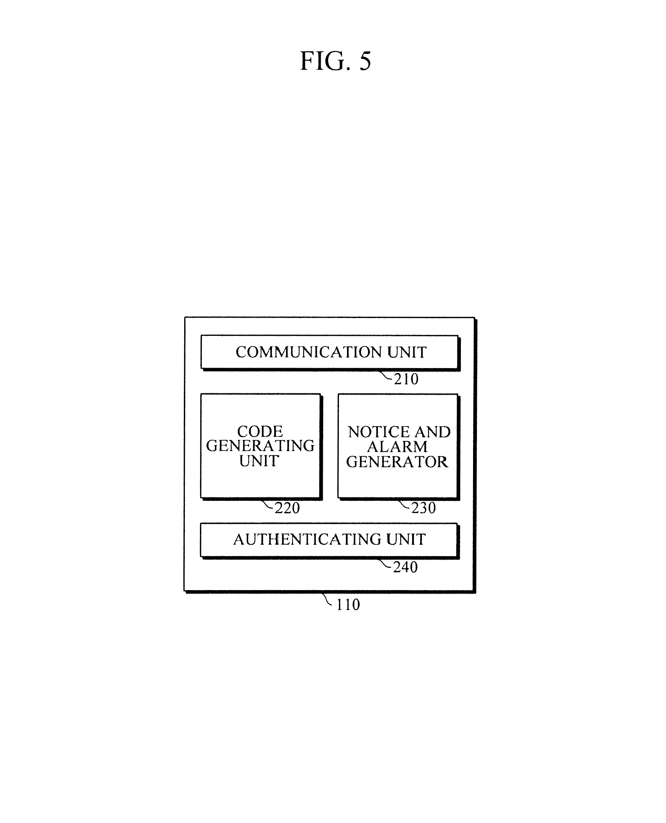

FIG. 5 illustrates another example embodiment of the IMD. Referring to the example embodiment illustrated in FIG. 5, the IMD 110 may further include the security engine 240 in addition to the communication module 210, the code processor/pattern store 220, and the notice and alarm generator 230 which are shown in the example embodiment illustrated in FIG. 3.

The operations of the communication module 210, the code processor/pattern store 220, and the notice and alarm generator 230 may be the same as those shown in the example embodiment illustrated in FIG. 3.

The IMD 110 may communicate with the external device 100 through the communication module 210, and the security engine 240 may verify whether the external device 100 is authorized to acquire state information when the external device 100 accessesthe IMD to request state information of the IMD 110 or physiological/pathological state information of the user.

A variety of methods may be utilized to verify the external device. For example, an identification stored in the external device may be matched with an identification stored in the IMD. Once the external device 100 has been verified, the notice and alarm generator 230 may generate a signal to notify the user of an event that the external device 100 accesses the IMD 110 and the user may make a specific movement in order to generate a motion code to allow the IMD to recognize user's intention.

The code processor/pattern store 220 may detect the movement and generate the motion code. The IMD 110 may determine whether to perform a specific activity based on the generated motion code, and may provide the information requested by the external device 100 or control other operation.

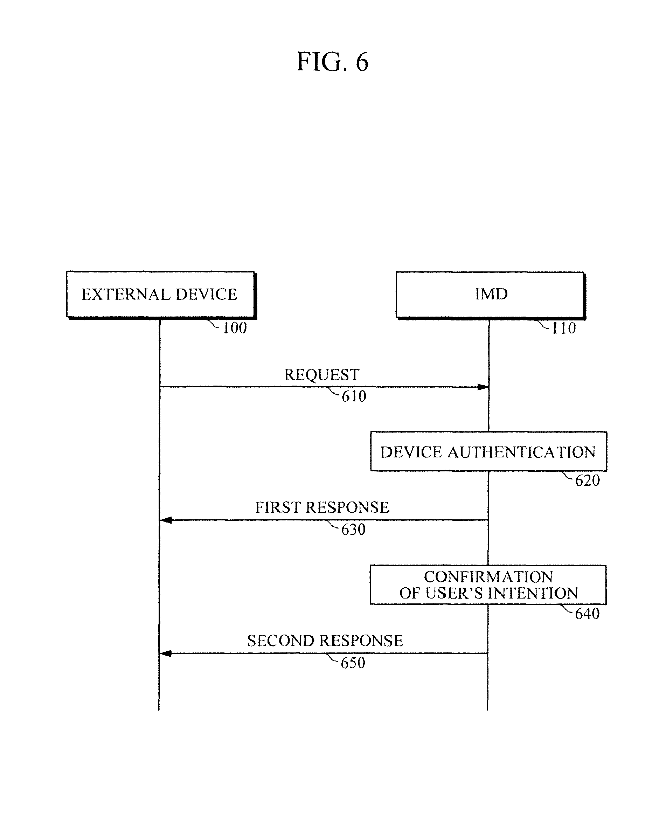

FIG. 6 illustrates a flowchart of another example embodiment of a method of controlling the IMD in response to an external device accessing the IMD.

The method shown in the example embodiment illustrated in FIG. 6 is implemented between the external device 100 and the IMD 110 based on their basic configurations.

First, the external device 100 accesses and requests the IMD 110, which may store state information of itself or physiological/pathological state information of a user, to provide such information (610).

Then, in response to the request, the IMD 110 may perform device authentication on the accessed external device 100 (620).

Once the device authentication has been completed between the IMD 110 and the external device 100, the IMD 110 may transmit a first response to the external device 100 to inform of the completion of the device authentication or provide the information requested (630). If the IMD 110 provides the requested information to the external device 100 based on the first response, the operation of the IMD 110 may be terminated.

After the completion of the device authentication, the IMD 110 may confirm with the user whether to provide the requested information to the external device (640).

When the user makes a specific movement, a motion code which allows the IMD 110 to recognize the user's intention may be generated and transmitted to the IMD 110. Accordingly, the IMD 110 may confirm the user's intention based on the motion code.

Then, the IMD 110 transmits a second response to the external device 100 to provide or reject the requested information based on the user's intention which has been confirmed at 640 (650).

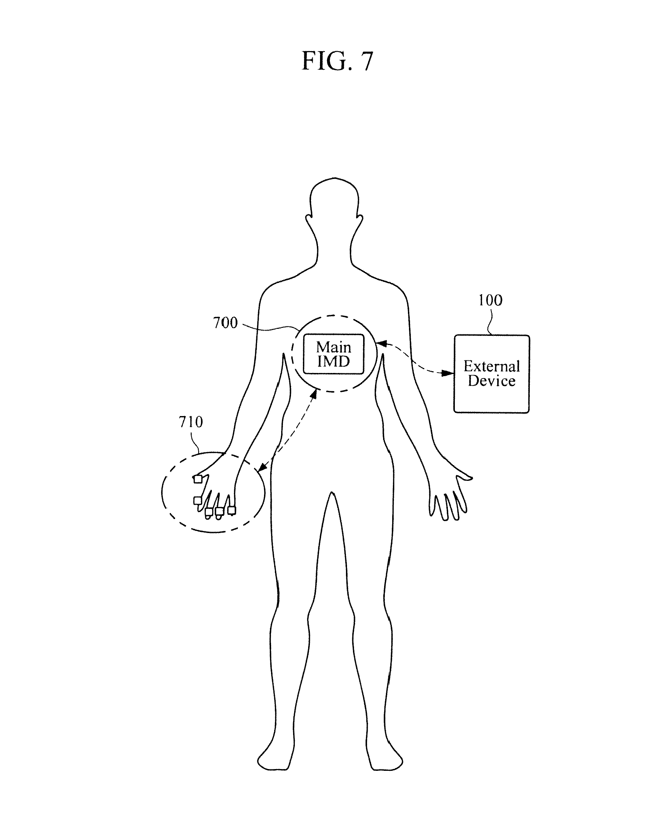

FIG. 7 illustrates an example embodiment of an IMD implanted in a human body.

Referring to the example embodiment illustrated in FIG. 7, the external device 100 may identify user's intention through a motion sensor 710 to control the IMD 700 when an external device 100 accesses the IMD 700.

The IMD 700 may include the configuration of the IMD shown in the example embodiment illustrated in FIG. 2.

Referring to the example embodiment illustrated in FIG. 7, the IMD 700 may include an information store, an information store access arbitrator, a medical sensor, and a main controller and these elements may have, respectively, the same functions as those shown in the example embodiment illustrated in FIG. 2.

In addition to the above elements, the IMD 700 may further include a communication module, a code processor/pattern store, and a notice and alarm generator 230.

The respective elements will be described in connection with a procedure of confirming the user's intention through the motion sensor 710.

The IMD 700 which may be implanted in a human body to collect physiological/pathological state information of a user and perform a medical procedure to normalize biological activities in the user's body and may communicate with the external device 100 (see FIG. 1) may include the communication module for the communication with the external device 100.

The external device 100 may be connected with the communication module of the IMD 700 and may acquire the state information of the IMD 700, or may access the IMD 700 to acquire the physiological/pathological information of the user stored in the IMD 700. In addition, the communication module may communicate with the motion sensor 710 and receive a motion state detected by the motion sensor 710, and thereby recognize the user's intention.

The notice and alarm generator 230 may generate a signal to inform the user that the IMD 700 is able to receive a motion code for recognizing the user's intention. Based on the motion code, the IMD 700 may be controlled to perform a specific activity.

In this case, the motion code may be dependent on the change in position of a part of a user's body based on user's motion state which includes 1) the standstill state and moving state of the user or 2) the moving state of user's pupils. The user's motion state may be detected by the motion sensor 710.

Although in the example embodiment illustrated in FIG. 7 the motion sensor 710 is located on user's fingers, the motion sensor 710 may be put or implanted in any part of the user's body as long as 1) the user can move the part as he/she wants and thereby generate a motion state (including a standstill state), and 2) the motion sensor 710 a) may detect the motion state resulting from the user's movement, and b) may acquire values related to changes in positions of the user's body.

In this case, the motion sensor 710 may use any method that adheres or fixes the motion sensor 710 on a body part (fingers in the example embodiment illustrated FIG. 7), and the motion sensor 710 may be in a wearable form (thimble-shaped in FIG. 7).

Further, the motion sensor 710 may not only be put or implanted in the user's body but also implemented as an individual device that is located outside of the user's body as long as it is capable of detecting a motion state of the user's body. In this regard, the motion sensor 710 implemented as an individual device may only need to detect the movement of the user from the outside the user's body and transmit the detected motion state to the IMD 700 implanted inside the user's body via any method.

Moreover, the implementation of the motion sensor 710 may not be limited to the implementations described. For example, there may be a single motion sensor to detect a simple movement or there may be a plurality of motion sensors in a form which allows identification of the user's intention based on combination of patterns and three-dimensional relations of a variety of movements.

The notice and alarm generator 230 may generate a signal to notify the user that the external device 100 attempts access to the IMD 700. In this case, the signal may be in any form including light, sound, vibration, and any kind of stimulus, as long as the user can recognize. In addition, the signal can be implemented as, for example, a clock, a terminal, or an element of an external device, based on any method as long as the user may recognize the signal.

The code processor/pattern store may receive a motion state of the user from the motion sensor 710 to control the IMD 700 which is implanted in the user's body, and may generate and store the motion code based on the motion state. In another example embodiment, the motion sensor 710 may detect motion state of the user and generate a motion code based on the detected motion state, and transmit the motion code to the IMD 700. In this case, the code processor/pattern store for generating a motion code may be included in the motion sensor instead of the IMD 700.

The IMD 700 may perform a specific operation based on user's intention identified from the motion code generated as described above.

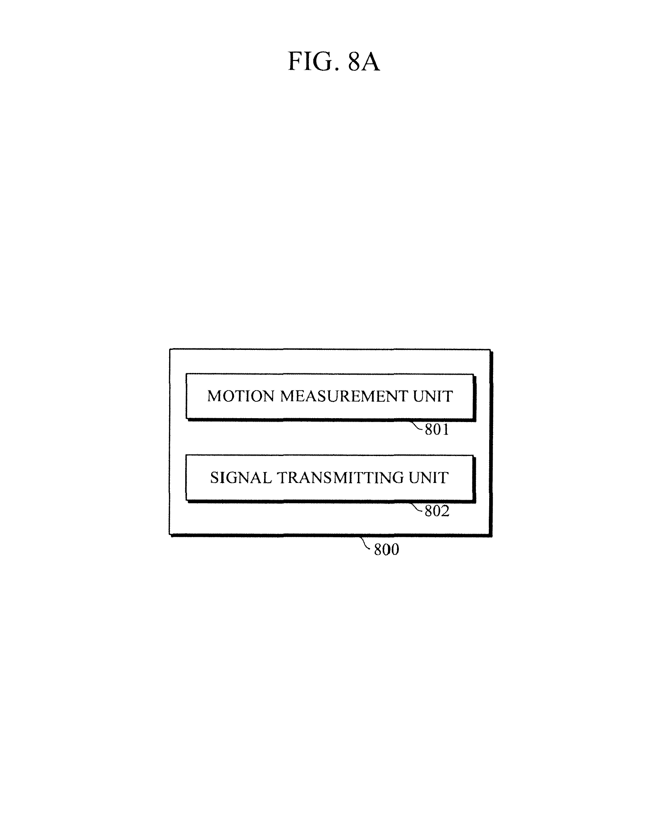

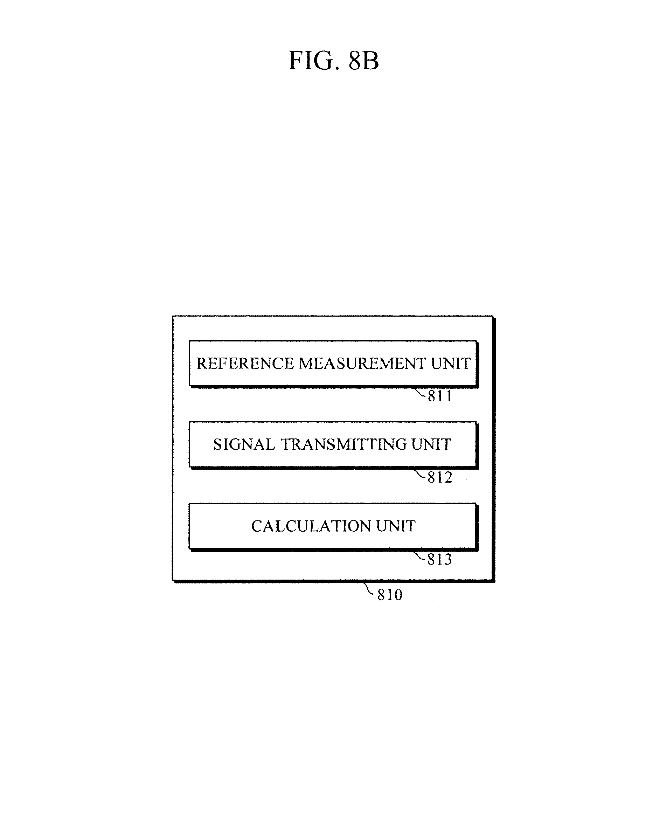

FIGS. 8A and 8B illustrate example embodiments of a motion sensor of an IMD.

The example embodiment illustrated in FIG. 8A shows a basic configuration of a first motion sensor 800. Referring to the example embodiment illustrated in FIG. 8A, the first motion sensor 800 may include a motion measurement unit 801 and a signal transmitting unit 802.

The motion measurement unit 801 may detect a movement of the user and measure a change in position of the user's body.

The signal transmitting unit 802 may communicate with the IMD 700 (refer to FIG. 7) and transmit the change in position which may be measured by the motion measurement unit 801 1 to the IMD 700.

The example embodiment illustrated in FIG. 8B shows a configuration of a second motion sensor 810 that detects a motion which is a reference motion from among motions detected by a plurality of motion sensors. Referring to the example embodiment illustrated in FIG. 8B, the second motion sensor 810 may include a reference measurement unit 811, a signal transmitting unit 812, and a calculation unit 813.

The reference measurement unit 811 may be the same as the motion measurement unit 801 in terms of a function that measures a change in position of a user's body by detecting a motion of the user.

The reference measurement unit 811 of the second motion sensor 810 may be different from the motion measurement unit 801 in that it may determine a reference motion for 1) collecting a plurality of changes in positions which are measured by a plurality of motion sensors having the same configuration as the first motion sensor 800 or 2) forming various forms of codes or patterns based on the plurality of changes in positions.

Thus, the reference measurement unit 811 may determine a motion of a specific part of the user's body as a reference motion, and the calculation unit 813 may calculate a current motion based on a relation between the reference motion determined by the reference measurement unit 811 and motions detected by a plurality of first motion sensors 800.

The signal transmitting unit 812 may 1) communicate with the IMD 700 and the plurality of first motion sensors 800, 2) receive the position changes measured by the first motion sensors 800, and 3) transmit the position changes to the IMD 700. The position changes may be transmitted through a general wireless communication method or through the body.

By the above procedures, various forms of motion codes which reflect diverse intentions of the user may be generated so that a diversity of control over the IMD 700 may be realized.

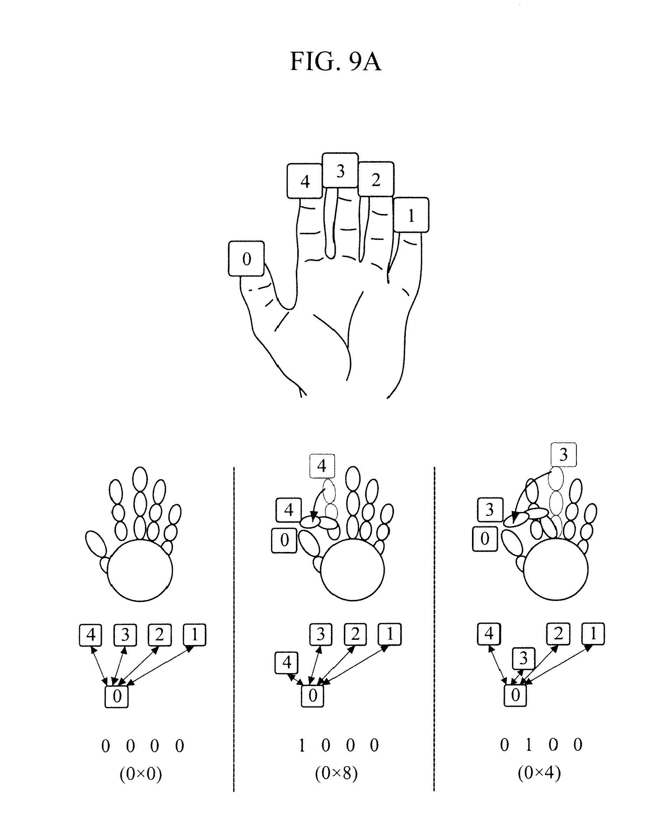

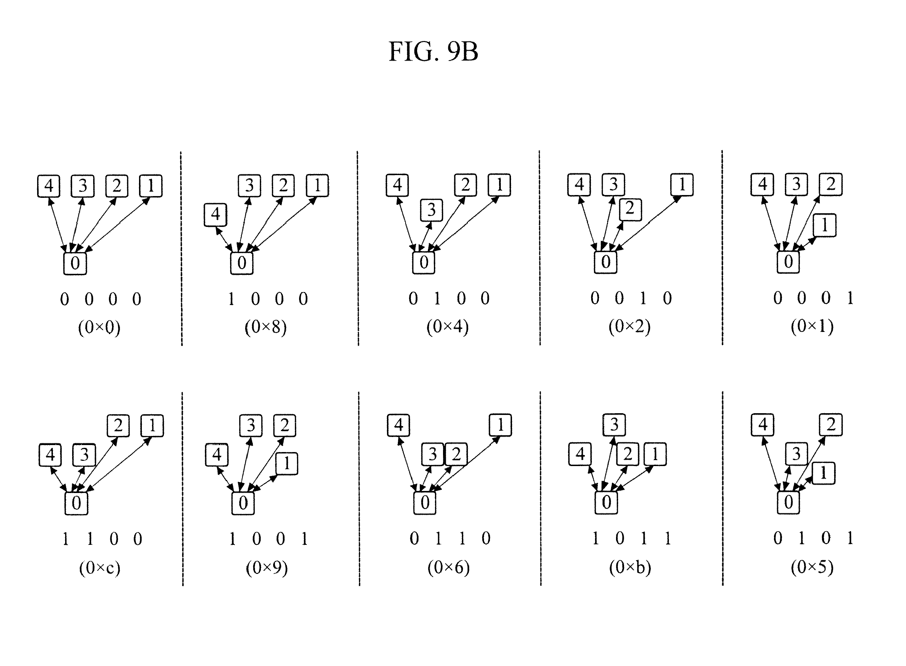

FIGS. 9A and 9B illustrate example embodiments for explaining motion codes generated by a plurality of motion sensors of an IMD.

As described above, the first motion sensor 800 (referring to FIG. 8A) and the second motion sensor 810 may be applied to any part of a user's body which may make a movement, and as shown in the example embodiment illustrated in FIG. 7, the first motion sensor 800 or the second motion sensor 810 may be worn on fingers. FIGS. 9A and 9B show how codes or patterns may be generated from changes in positions of fingers.

As illustrated in FIG. 9A, a motion sensor #0 may be adhered to or implanted in the thumb, a motion sensor #4 may be adhered to or implanted in the index finger, a motion sensor #3 may be adhered to or implanted in the middle finger, a motion sensor #2 may be adhered to or implanted in the ring finger, and a motion sensor #1 may be adhered to or implanted in the little finger.

In this case, it may be assumed that the motion sensor #0 is applied with the second motion sensor 810 as shown in the example embodiment illustrated in FIG 8B, and the rest of the motion sensors are applied with the first motion sensors as shown in the example illustrated in FIG. 8A. Under such assumption, a position change of each finger may be detected based on the movement of a hand on the basis of the thumb as a reference and the position change may be mapped as described below.

In the example embodiment illustrated in FIG. 9A, a state in which the thumb is away from the fingers (or the thumb and the fingers spread away from each other) may be mapped to a value '0000' (0x0), a state in which only the index finger is bent toward the thumb and the rest fingers do not make significant movement may be mapped to a value '1000' (0x8), and a state in which the middle finger is bent toward the thumb and the rest fingers do not make notable movement may be mapped to a value '0100' (0x4).

In addition, the example embodiment illustrated in FIG. 9B may be based on an assumption that motion sensors #0, #4, #3, #2, and #1 are, respectively, adhered to or implanted in the thumb to the little fingers in this order, and shows various motion codes generated according to finger movements.

In other cases, the same motions may be mapped to codes of different values, and the mapping may be implemented in different ways.

The example embodiments illustrated in FIGS. 9A and 9B may show motion codes or patterns of simple formats which are generated by the motion sensors adhered to or implanted in the five fingers and, in a similar manner, mapping may be performed with respect to other parts of the user's body by utilizing motion state generated.

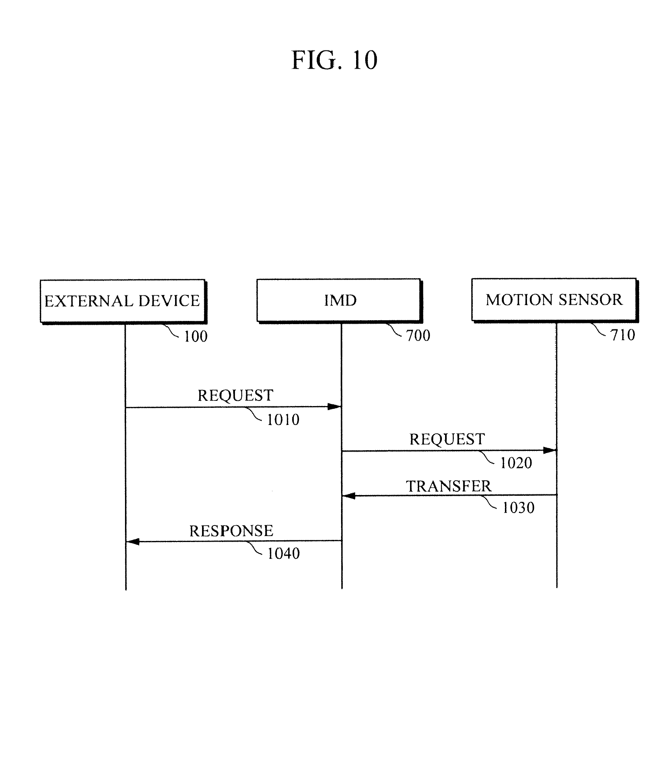

FIG. 10 illustrates a flowchart of an example embodiment of a method of controlling an IMD having a motion sensor.

The method shown in FIG. 10 may be implemented by an external device 100, an IMD 700, and the motion sensor 710, and will be described in relation with the example embodiment illustrated in FIG. 7.

In the example embodiment illustrated in FIG. 10, the IMD 700 may include a communication module, a code processor/pattern store, and a notice and alarm generator as shown in the example embodiment illustrated in FIG. 3.

The external device 100 may request the IMD 700, which may retain state information of itself or physiological/pathological state information of a user, to transmit the state information (1010) to the external device.

The IMD 700 that has received the request for the state information may confirm with the user whether to provide the state information to the external device 100 (1020).

The notice and alarm generator may generate a signal to notify the user that the external device 100 attempts access to the IMD 700. In this case, the signal may be in any form including light, sound, vibration, and any kind of stimulus, as long as the user can recognize the signal.

In this case, the notice and alarm generator of the IMD 700 may draw attention or make the user recognize the access of the external device 100 by use of a signal, which is in the form of light, sound, vibration, or any kind of stimulus. In response to the signal, the user 1) may recognize an inquiry whether to provide the requested information, 2) may determine whether to provide the state information and 3) may transmit the determination result to the IMD 700 through the motion sensor 710.

That is, the motion sensor 710 may detect a position change resulting from the motion of the user, and may transfer the detected result to the IMD 700 (1030). The IMD 700 may receive the position change of a motion and may determine whether to provide the requested state information to the external device 100.

If the user determines to provide the state information to the external device 100 (if an identified intention of the user indicates approving the providing of the requested state information), the state information retained by the IMD 700 may be transmitted to the external device 100 (1040). If the user rejects the request to provide the state information (if an identified intention of the user indicates rejecting the providing of the requested state information), the IMD 700 may transmit a refusal response to the external device 100 (1040).

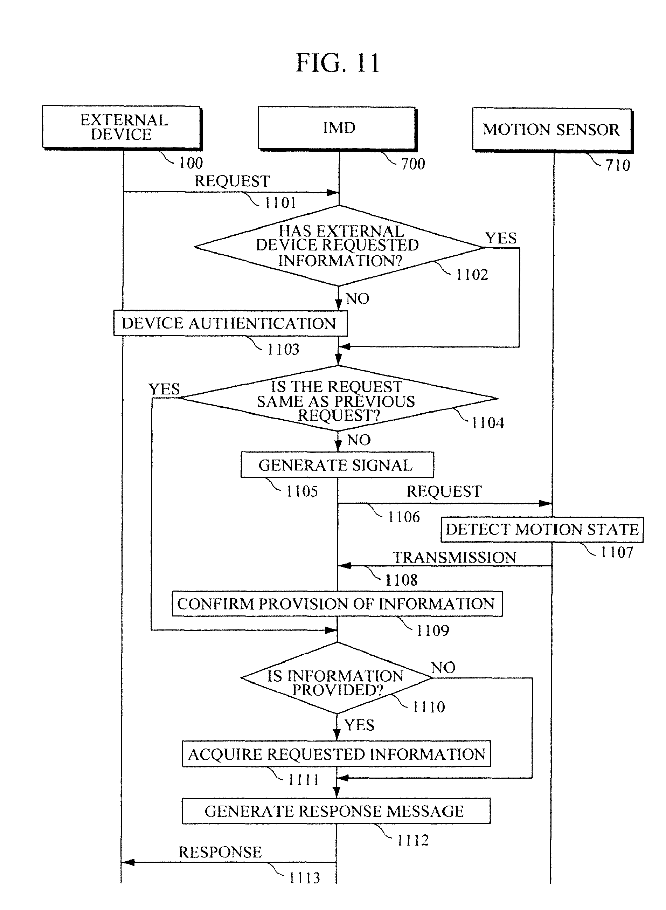

FIG. 11 illustrates a method of controlling the IMD having a motion sensor.

Similar to the example embodiment illustrated in FIG. 10, the example embodiment illustrated in FIG. 11 may be implemented by an external device 100, an IMD 700, and a motion sensor 710.

However, the IMD 700 shown in the example embodiment illustrated in FIG. 11 may further include a security engine in addition to a communication module, a code processor/pattern store, and a notice and alarm generator as included in the IMD 700 shown in the example embodiment illustrated in FIG. 10.

The method shown in the example embodiment illustrated in FIG. 10 may be implemented by the IMD including the communication module 210, the code processor/pattern store 220, and the notice and alarm generator 230 as shown in the example embodiment illustrated in FIG. 3, and the method shown in the example embodiment illustrated in FIG. 11 I may be implemented by the IMD including the communication module 210, the code processor/pattern store 220, the notice and alarm generator 230, and the security engine 240 as shown in the example embodiment illustrated in FIG. 5.

The external device 100 may request the IMD 700, which may retain state information of itself or physiological/pathological state information of a user, to transmit the state information (1101).

Then, the IMD 700 may check whether the IMD has previously received a request to transmit the state information from the external device 100 (1102), and if the IMD 700 has not received a request from the same external device 100, device authentication is carried out between the external device 100 and the IMD 700 (1103). If the external device 100 is not able to complete the device authentication because the external device 100 is not authorized to be provided with the state information, the IMD 700 may transmit a refusal response to the external device 100.

In response to the request for the state information being previously received from the same external device 100 or the device authentication has been completed at 1103, it may be determined whether the requested state information is provided according to predefined access control policies or the providing of the state information to the external device 100 is determined through the motion sensor 710 (1104).

The access control policies may be implemented in various ways based on the level of privacy and the level of security requested by the user for the state information retained in the IMD 700. In the example embodiment illustrated in FIG. 11, if the IMD 700 has received a request for the state information from the external device 100, the access control policies may be implemented for the state information retained in the IMD 700 to be transmitted directly to the external device 100 (1113) 1) when the access is carried out within an available period predefined for the external device 100 (or the device authentication of the external device 100 has been completed) or 2) when the currently requested state information is the same as the previously requested state information (1104). The confirmation of providing of the state information may be carried out via the motion sensor 710.

Subsequently, to confirm a user's intention of whether to provide the requested state information through the motion sensor 710, the IMD 700 may generate a signal for confirmation of the user (1105), and may request the user to confirm whether to provide the requested state information to the external device 100 (1106).

The user may be enabled to recognize the request for the state information from the external device 100 through the signal generated at 1105, and makes a motion as described above. Then, the motion state may be detected by the motion sensor 710 (1107). In this case, the motion sensor 710 may compute the motion state or a motion code based on a standstill state and moving state of the user or a moving state of user's pupils (1107), and the motion state may allow the recognition of the user's intention for the request for the providing of the state information of the IMD 700. The motion state may be implemented as a code or a pattern, as described above.

The computed motion state or motion code may be transmitted to the IMD 700 (1108). The IMD 700 may receive the user's motion state from the motion sensor 710, and may identify the user's intention of whether to provide the requested state information to the external device 100(1109).

If the user authenticates the providing of the state information (1110), that is, if the requested state information is determined to be provided to the external device 100, the requested state information retained by the IMD 700 may be transmitted to the external device 100 (1111, 1112, and 1113). If the user refuses to approve the providing of the state information (1110), that is, if the requested state information is determined not to be provided to the external device 100, the IMD 700 may generate a response message to refuse to provide the state information to the external device 100 (1112) and may transmit the generated response message to the external device 100 (1113).

The above-described IMD may allow the recognition of the user's intention with respect to an access or a request for information by an external device, and may be controlled by the recognized user's intention. Hence, the recognition of the user's intention may be included as a technical element.

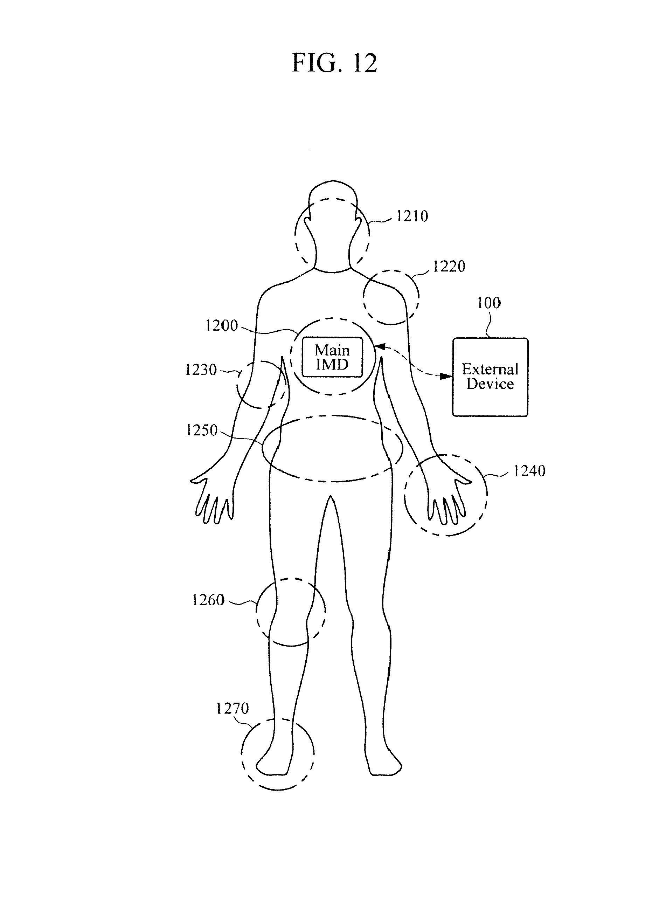

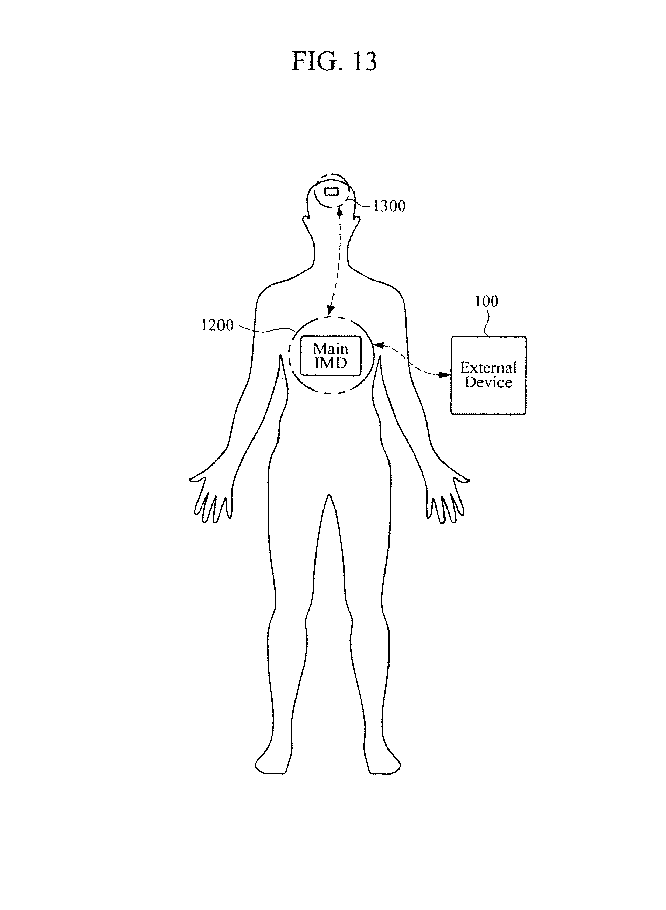

Accordingly, example embodiments of recognition of the user's intention, as illustrated in FIGS. 12 and 13, may be taken into consideration. However, any other example embodiments which may recognize a user's intention falls within the scope of the teachings described herein.

FIG. 12 illustrates an example embodiment showing body parts of a user that allows the recognition of a user's intention through a motion state of the user.

Referring to the example embodiment illustrated in FIG. 12, an implantable medical device (IMD) 1200 may have a structure to recognize the user's intention for an access or an information request from an external device 100, and be controlled by the recognized intention.

As shown in the example illustrated in FIG. 12, the body parts that allow the recognition of the user's intention may include a head 1210 (facial muscles, eyes, pupils, eyelids, ears, neck, and the like), shoulders 1220, elbows 1230, hands or wrists 1240, a waist 1250, knees 1260, feet or ankles 1270 and the like.

The motion sensor illustrated in the example embodiment of FIG. 8A or 8B may be put or implanted in any body part to detect the motion state, thereby recognizing the user's intention. In addition, a third device which has been previously described in the example embodiment illustrated in FIG. 7 may be employed as long as the device can detect the motion state for recognizing the user's intention.

Further, the user's intention may be based on consciousness (thinking) resulting from a brain activity, and any form of directly recognizing the user's intention resulting from the brain activity falls within the scope of the teachings described herein.

FIG. 13 illustrates an example embodiment of a structure for controlling an IMD by recognizing a user's intention through the direct detection of the brain activity.

Referring to the example embodiment illustrated in FIG. 13, the IMD 1200 1) may recognize the user's intention with respect to an access or an information request from an external device 100 using a detecting unit 1300 implemented in a user's brain or in proximity to the brain, and 2) be controlled based on the recognized intention.

The IMD 1200 1) which may be implanted in a human body to collect physiological/pathological state information of a user or 2) which may be closely connected to the organs of the users to control the operation of the organs may have the same structure as shown in the example embodiment illustrated in FIG. 2.

Hence, the IMD 1200 may include an info store, an info store access arbitrator, a medical sensor, and a main controller, and their functions may be substantially the same as those shown in the example embodiment illustrated in FIG. 2.

In addition to the above primary structure, the IMD 1200 may further include a communication module, a code processor/pattern store, a notice and alarm generator, and a security engine. The code processor/pattern store of the example embodiment illustrated in FIG. 13 may be partially or entirely substituted by the detecting unit 1300 which may be implemented in the user's brain or in proximity to the brain.

The external device 100 may be connected to the communication module of the IMD 1200 1) to acquire state information of the IMD 1200, or 2) to access the IMD 1200 to acquire physiological/pathological information of the user which may be stored in the IMD 1200.

The IMD 1200 may be enabled to recognize the user's intention through the detecting unit 1300 with respect to a request of the external device 100 that accesses the IMD 1200 for various purposes.

The detecting unit 1300 may be directly or indirectly connected to the user's brain to detect movements (gestures or motions) of the user, including 1) a standstill state or moving state of the user and a movement of pupils, through an electrical reaction in a motor nerve or a sensory nerve, or to directly analyze or 2) identify the user's consciousness based on a brainwave state resulting from an electrical/chemical reaction in the user's brain. Further, the detecting unit 1300 may generate a motion code based on the detected or analyzed result to allow the IMD 1200 to reflect the user's intention with respect to the access from the external device 100.

Thus, the IMD 1200 may receive the motion code generated by the detecting unit 1300 which directly/indirectly may recognize the user's intention resulting from the brain activity, and may provide the requested state information, may refuse to provide, or may provide any other response to the external device 100 based on the received motion code.

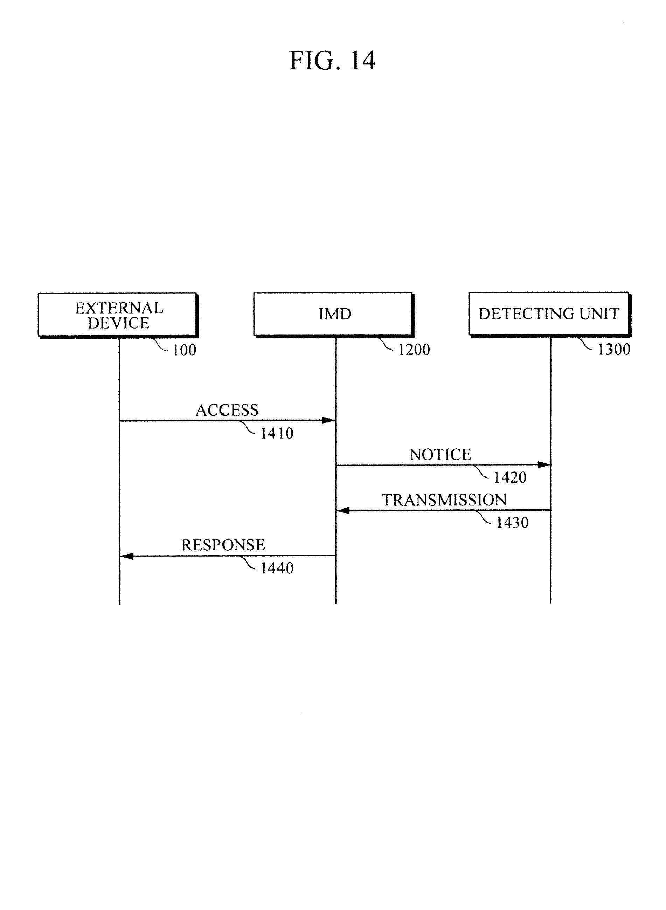

FIG. 14 illustrates a flowchart of an example embodiment of a method of controlling an implantable medical device (IMD) by recognizing a user's intention based on direct detection of brain activity.

The method of controlling the IMD may be performed between an external device 100, the IMD 1200, and a detecting unit 1300. The method illustrated in FIG. 14 may control the IMD 1200 having the primary structure which is shown in the example embodiment illustrated in FIG. 13.

The external device 100 may access the IMD 110, which may retain state information of itself or physiological/pathological state information of a user, to acquire the state information (1410).

The IMD 1200 may confirm the user whether to allow access of the external device 100 or to provide the requested state information. In this case, the IMD 1200 may generate a signal in the form of light, sound, vibration, or any kind of stimulus to notify the user that the external device 100 attempts access or issues a request to the IMD 1200 (1420).

Then, the user may recognize an inquiry whether to allow the providing of the requested state information or the access of the external device 100 through the signal, may make a decision about the provision or the state information or the access of the external device 100, and may transmit the result to the IMD 1200 through a motion code generated by the detecting unit 1300 (1430).

The detecting unit 1300 may be directly or indirectly connected to the user's brain 1) to detect movements (gestures or motions) of the user, including a standstill state or moving state of the user and a movement of pupils, through an electrical reaction in a motor nerve or a sensory nerve, or 2) to directly analyze or identify the user's consciousness based on a brainwave state resulting from an electrical/chemical reaction in the user's brain. Further, the detecting unit 1300 may generate a motion code based on the detected or analyzed result to allow the IMD 1200 to reflect the user's intention with respect to the access from the external device 100.

Thus, the IMD 1200 may receive the motion code generated by the detecting unit 1300 which directly/indirectly recognizes the user's intention resulting from the brain activity, and may provide the requested state information, refuse to provide, or may provide any other response to the external device 100 according to the received motion code (1440). The IMD 1200 1) may be allowed to recognize the user's intention based on the user's motion state including a standstill state and a moving state of the user, a moving state of pupils, and a brainwave state, and 2) may determine whether to permit the access of the external device 100 according to the user's intention.

When the user confirms to provide information (in this example, in which the user only thinks of allowing the provision of information), or when the IMD 1200 provides the requested state information, the IMD 1200 may transmit the requested information to the external device 100 (1440). When the user refuses to provide the information (in this example embodiment, when the user thinks of refusing to provide the information), the IMD 1200 may transmit a refusal response to the external device 100 (1440).

Program instructions to perform a method described herein, or one or more operations thereof, may be recorded, stored, or fixed in one or more computer-readable storage media. The program instructions may be implemented by a computer. For example, the computer may cause a processor to execute the program instructions. The media may include, alone or in combination with the program instructions, data files, data structures, and the like. Examples of computer-readable media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The program instructions, that is, software, may be distributed over network coupled computer systems so that the software is stored and executed in a distributed fashion. For example, the software and data may be stored by one or more computer readable recording mediums. Also, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein. Also, the described unit to perform an operation or a method may be hardware, software, or some combination of hardware and software. For example, the unit may be a software package running on a computer or the computer on which that software is running. A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components. Accordingly, other implementations are within the scope of the following claims.

## Claims

1. An implantable medical device, IMD, comprising:
a medical procedure performing unit configured to perform a medical procedure inside a body of a user;
a code generating unit configured to detect a motion state of the user, and to generate a motion code that represents a user's intention based on the detected motion state; and
a control unit configured to control the medical procedure of the medical procedure performing unit based on the generated motion code.

2. The IMD of claim 1, wherein the medical procedure performing unit is adapted to collect physiological/pathological state information of the user, and wherein the control unit is adapted to determine whether to transmit the physiological/pathological state information to an external device based on the motion code.

3. The IMD of claim 1 or 2, wherein the code generating unit is adapted to detect the motion state that includes a standstill state of the user, a moving state of the user, a moving state of user's pupils, a brainwave state of the user, or a combination thereof.

4. The IMD of any one of claims 1 to 3, wherein the code generating unit comprises a first sensor configured to set a reference for the motion state of the user and a second sensor configured to detect the motion state which includes the standstill state of the user, a moving state of the user, a moving state of the user's pupils, or a combination thereof, wherein the first sensor or the second sensor is placed outside of the user's body to detect a motion state of the user.

5. The IMD of any one of claims 1 to 4, wherein the code generating unit comprises a sensor configured to be worn on or implanted in a user's finger to detect the motion state of the user.

6. The IMD of claim 2, further comprising:
an authenticating unit configured to authenticate whether the external device which communicates with the IMD is authorized to acquire the physiological/pathological state information of the user, wherein the code generating unit is further adapted to generate the motion code in response to the authenticating unit authenticating the external device to be authorized to acquire the physiological/pathological state information, and/or
wherein the IMD further comprises a notice and alarm generator configured to generate a signal to notify of an access of the external device in response to the external device attempting to access the IMD to communicate therewith, wherein the notice and alarm generator is adapted to generate a recognizable signal in the form of light, sound, vibration, stimulus, or a combination thereof.

7. The IMD of any one of claims 1 to 6, wherein the user's intention includes allowing the performance of the medical procedure or restricting the performance of the medical procedure.

8. An implantable medical device, IMD, comprising:
an information collecting unit configured to collect physiological/pathological state information of a user; and
a provision determining unit configured to detect a motion state of the user, and to determine whether to provide the physiological/pathological state information to an external device according to a user's intention identified based on the detected motion state.

9. The IMD of claim 8, wherein the provision determining unit comprises:
a communication module configured to transmit the physiological/pathological state information to the external device;
a detecting unit configured to detect a motion state which includes a standstill state of the user, a moving state of the user, a moving state of user's pupils, a brainwave state of the user, or a combination thereof;
a code generating unit configured to generate a motion code that represents the user's intention for the providing of the physiological/pathological state information based on the detected motion state; and
a control unit configured to control the communication module based on the generated motion code,
wherein the code generating unit is adapted to compare the motion state of the user with a previously stored motion state of the user and to generate a motion code corresponding to the detected motion state.

10. The IMD of claim 8 or 9, further comprising:
an authenticating unit configured to authenticate whether the external device that communicates with the IMD is authorized to acquire the physiological/pathological state information, wherein the authenticating unit is adapted to authenticate the external device by determining whether the external device has previously accessed or whether physiological/pathological state information to be transmitted is the same as the physiological/pathological state information requested by the external device, or wherein the provision determining unit is adapted to detect the motion state of the user in response to the external device being authorized to acquire the physiological/pathological state information.

11. A method of controlling an implantable medical device, IMD, with respect to an access of an external device, the method comprising:
receiving a request for state information of the IMD or physiological/pathological state information of a user from the external device;
identifying a user's intention by detecting a motion state of the user; and
determining whether to transmit the requested state information to the external device according to the identified user's intention.

12. The method of claim 11, wherein the identifying of the user's intention comprises detecting the motion state of the user which includes a standstill state of the user, a moving state of the user, a moving state of user's pupils, a brainwave state of the user, or a combination thereof, wherein the identifying of the user's intention comprises:
generating a motion code that represents a user's intention based on the detected motion state of the user; and
identifying the user's intention that corresponds to the generated motion code by comparing the generated motion code with a previously stored motion code.

13. The method of claim 11 or 12, wherein the determining whether to transmit the requested state information comprises providing the state information to the external device or transmitting a refusal response to the external device according to the identified user's intention.

14. An external device for communicating with an implantable medical device, IMD, the external device comprising:
a communications unit configured to access state information stored in the IMD; and
a reception unit configured to receive the state information determined by a motion state of the user corresponding to a user's intention detected by the IMD.

15. The external device of claim 14, wherein the user's intention includes allowing the transmission of the state information or restricting the transmission of the state information.
